(19) Europäisches Patentamt
European Patent Office
Office européen des brevets

(11) **EP 2 809 401 B1**

(12) **EUROPÄISCHE PATENTSCHRIFT**

(45) Veröffentlichungstag und Bekanntmachung des Hinweises auf die Patenterteilung:
**29.03.2017 Patentblatt 2017/13**

(21) Anmeldenummer: **13701293.6**

(22) Anmeldetag: **29.01.2013**

(51) Int Cl.:
*A61Q 5/08* (2006.01)     *A61K 8/73* (2006.01)
*A61K 8/23* (2006.01)

(86) Internationale Anmeldenummer:
**PCT/EP2013/051676**

(87) Internationale Veröffentlichungsnummer:
**WO 2013/113694 (08.08.2013 Gazette 2013/32)**

(54) **REDUKTIVER FARBABZUG**

REDUCTIVE COLOR REMOVAL

DÉCOLORATION PAR RÉDUCTION

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **30.01.2012 DE 102012201265**

(43) Veröffentlichungstag der Anmeldung:
**10.12.2014 Patentblatt 2014/50**

(73) Patentinhaber: **Henkel AG & Co. KGaA
40589 Düsseldorf (DE)**

(72) Erfinder: **GOUTSIS, Konstantin
41363 Jüchen (DE)**

(56) Entgegenhaltungen:
| | |
|---|---|
| EP-A2- 1 300 136 | DE-A1- 2 426 055 |
| DE-A1- 2 628 903 | DE-A1-102006 053 343 |
| DE-A1-102006 053 402 | DE-A1-102008 034 845 |

Bemerkungen:
Die Akte enthält technische Angaben, die nach dem Eingang der Anmeldung eingereicht wurden und die nicht in dieser Patentschrift enthalten sind.

EP 2 809 401 B1

**Beschreibung**

Die Erfindung betrifft ein Mittel und Verfahren zur reduktiven Entfärbung von oxidativ gefärbten keratinischen Fasern, insbesondere menschlichen Haaren.

Für das Färben von keratinhaltigen Fasern, insbesondere in der kosmetischen Färbung menschlicher Haare, werden im Allgemeinen entweder Färbesysteme auf Basis von direktziehenden Farbstoffen oder Oxidationsfarbstoffen eingesetzt. Oxidationsfarbstoffe entstehen durch oxidative Kupplung einer oder mehrerer Entwicklerkomponenten untereinander oder mit einer oder mehreren Kupplerkomponenten. Kuppler- und Entwicklerkomponenten werden auch als Oxidationsfarbstoffvorprodukte bezeichnet. Die oxidative Kupplung findet üblicherweise während des Färbevorgangs statt, so dass die Farbstoffvorprodukte in das Substrat hinein diffundieren können und der Farbstoff sich in dem Substrat bildet. Dadurch wird die Echtheit der Färbungen verbessert, da beispielsweise das Auswaschen aus dem Substrat erschwert ist.

Aus modischen Gründen, aber auch bei unerwünschten Färbeergebnissen oder falscher Nuancenauswahl, kann jedoch der Bedarf bestehen, oxidativ gefärbtes Haar in seinen farblichen Urzustand zurück zu versetzten. Zur Entfärbung von oxidativ gefärbten Haaren sind dem Fachmann sowohl oxidative als auch reduktive Entfärbemethoden bekannt.

Eine oxidative Entfärbung führt meist zu guten Ergebnissen. Oxidative Methoden benötigen üblicherweise die Anwendung von Oxidationsmitteln bei stark alkalischen pH-Werten. Durch die starke Oxidationswirkung des zur Entfärbung benutzten Oxidationsmittels kann die Struktur des Substrats chemisch verändert werden. Dies geht mit einer unerwünschten physikalischen Änderung des Substrats einher. Solchermaßen entfärbte Haare neigen zu Spliss-Bildung, können spröde werden oder insbesondere bei mehrmaliger Entfärbung gar brechen. Weiterhin kann es bei der oxidativen Entfärbungen zur deutlichen Farbverschiebungen, insbesondere in Richtung warmer gelb-orange bis rötlicher Töne, kommen, die in der Regel vom Verbraucher nicht erwünscht sind. Der visuelle Eindruck, die Haptik aber auch die Haltbarkeit des Substrats können durch solche oxidativen Methoden negativ beeinflusst werden. Es besteht daher das Bedürfnis, weniger schädigende Entfärbemethoden für oxidativ gefärbte Haare zu entwickeln.

Weniger Einfluss auf die Substratstruktur, insbesondere auf die Struktur keratinhaltiger Fasern, nehmen reduktive Entfärbemittel. Die reduktiven Entfärbemittel entfärben kaum die Naturhaarfarbe, sondern wirken lediglich auf die durch Färbung aufgetragenen Farbstoffe reduktiv ein. Es tritt somit kaum eine Aufhellung des Haars auf. Als Reduktionsmittel werden üblicherweise reduktiv wirkende Schwefelverbindungen, wie Sulfite, Dithionite ($S_2O_4^{2-}$) oder Derivate der 1-Hydroxymethylsulfinsäure bzw. der 1-Aminomethylsulfinsäure, eingesetzt.

EP 1 300 136 A2 offenbart farbverändernde Mittel auf Basis von Dithionitsalzen. Im Beispielteil offenbart D1 farbverändernde Mittel, welche durch Vermischen von den Zusammensetzungen erhalten werden.

DE 10 2006 053 402 A1 betrifft Mittel zum reduktiven Farbabzug, welche ein Reduktionsmittel (auch Dithionitsalze) und ein Adsorptionsmittel enthalten

Aus DE 10152940 A sind reduzierende 2-Komponenten-Systeme bekannt, bei denen Dithionite in wässrigen Lösungen bei neutralen oder alkalischen pH-Werten zur Entfärbung eingesetzt werden. Deren Aufhellleistung ist jedoch begrenzt. Aus DE 102006053343 A1 sind Entfärbezubereitungen mit pH 1,5 auf Basis von Natriumdithionit bekannt, die unter Bestrahlung eingesetzt werden. Problematisch ist hierbei die Disproportionierung der freien dithionigen Säure ($H_2S_2O_4$) aus den angesäuerten Salzen zu Hydrogensulfit und Thiosulfat, welche eine verringerte Reduktionskraft besitzen. Daher bedürfen diese reduktiven Entfärbemittel einer Verbesserung der Entfärbeleistung. Substrate, die mit den Sulfinsäurederivaten des Standes der Technik reduktiv entfärbt wurden, erfahren einige Zeit nach dem Farbabzug eine unerwünschte Nachdunkelung. Ferner wünscht sich der Fachmann eine Verkürzung der Einwirkungsdauer des reduktiven Farbabzugsmittels.

Für eine leistungsstarke und gleichmäßige Entfärbung ist es erforderlich, dass das Entfärbemittel während des Entfärbevorgangs am Wirkort verbleibt und nicht aus den Fasern verläuft. Hierzu werden solche Mittel entsprechend verdickt und durch die erhöhte Viskosität der Mittel am Wirkort gehalten. Nachteilig bei der Verdickung ist jedoch, dass solche Systeme häufig opak und optisch wenig durchlässig sind.

Entscheidend für die Entfärbung der Fasern zum farblichen Urzustand ist jedoch die Möglichkeit, den Entfärbevorgang auf der Fasern visuell verfolgen zu können, um den geeigneten Zeitpunkt für die Beendung der Entfärbereaktion zu finden. Hierzu ist es besonders wünschenswert, dass die auf die Fasern aufgebrachten Entfärbezubereitungen trotz ausreichender Viskosität transparent sind, so dass die Farbänderung unmittelbar bestimmbar ist.

Aufgabe der vorliegenden Erfindung ist es, in der Entfärbekraft verbesserte reduktive Entfärbemittel und Entfärbemethoden bereitzustellen, die Haut und/oder keratinhaltige Fasern, insbesondere menschliches Haar, zum farblichen Ursprungszustand entfärben. Insbesondere soll der Entfärbevorgang dabei visuell verfolgt werden können, um den Endpunkt des Vorgangs ermitteln zu können. Die Substratstruktur soll dabei möglichst geschont werden. Ferner sollten die in den Entfärbemitteln eingesetzten Reduktionsmittel für eine kosmetische Verwendung physiologisch verträglich und toxikologisch unbedenklich sein.

Überraschenderweise wird die Aufgabe durch ein saures Mittel gelöst, welches unmittelbar vor der Anwendung aus einer sauren, wässrigen oder wässrig alkoholischen Zubereitung und einer weiteren Zubereitung, enthaltend Dithionitsalze hergestellt. Dadurch war es möglich, die bekannten Probleme der Instabilität von Dithioniten in sauren Milieu und der verringerten Entfärbeleistung bei neutralen und alkalischen pH-Werten zu lösen. Die Leistung der kosmetischen Entfärbung von keratinhaltigen Fasern, insbesondere menschlichem Haar, wird nachweislich erhöht und die Ursprungsfarbe lässt sich wieder herstellen. Weiterhin weisen die so behandelten Fasern einen verringerten Schädigungsgrad auf und der Vorgang lässt sich direkt nachverfolgen.

Ein erster Gegenstand der vorliegenden Erfindung ist daher ein Mittel zum Farbabzug von oxidativ gefärbten keratinischen Fasern, welches unmittelbar vor der Anwendung durch Mischen einer ersten wässrigen oder wässrig-alkoholischen Zubereitung (I), enthaltend mindestens eine mineralische Säure ausgewählt aus Salzsäure und/oder Schwefelsäure, mit einer zweiten Zubereitung (II), enthaltend mindestens ein Dithionitsalz, erhalten wird, dadurch gekennzeichnet, dass das anwendungsbereite Mittel einen pH-Wert von kleiner als 5,0 aufweist und zusätzlich mindestens einen Verdickungsmittel enthält.

Unter Keratinfasern oder keratinischen Fasern sind dabei Pelze, Wolle, Federn und insbesondere menschliche Haare zu verstehen.

Oxidativ gefärbte keratinische Fasern sind dabei Fasern, welche mit Oxidationsfarbstoffvorprodukten unter oxidativen Einfluss gefärbt wurden. Als übliche Oxidationsmittel können insbesondere Wasserstoffperoxid oder dessen Anlagerungsprodukte an Harnstoff, Melamin oder Natriumborat verwendet werden. Die Oxidation mit Luftsauerstoff als einzigem Oxidationsmittel kann allerdings bevorzugt sein. Weiterhin ist es möglich, die Oxidation mit Hilfe von Enzymen durchzuführen, wobei die Enzyme sowohl zur Erzeugung von oxidierenden Per-Verbindungen eingesetzt werden als auch zur Verstärkung der Wirkung einer geringen Menge vorhandener Oxidationsmittel. Zweckmäßigerweise wird die Zubereitung des Oxidationsmittels dann unmittelbar vor dem Färben der Haare mit der Zubereitung mit den Farbstoffvorprodukten vermischt. Das dabei entstehende gebrauchsfertige Haarfärbepräparat sollte bevorzugt einen pH-Wert im Bereich von 6 bis 11 aufweisen. Besonders bevorzugt ist die Anwendung der Haarfärbemittel in einem schwach alkalischen Milieu. Die Anwendungstemperaturen können in einem Bereich zwischen 15 und 40 °C, bevorzugt bei der Temperatur der Kopfhaut, liegen. Nach einer Einwirkungszeit von ca. 5 bis 45 min, insbesondere 15 bis 30 min, wird das Haarfärbemittel durch Ausspülen von dem zu färbenden Haar entfernt. Das Nachwaschen mit einem Shampoo entfällt, wenn ein stark tensidhaltiger Träger, z. B. ein Färbeshampoo, verwendet wurde.

Die eigentlichen Oxidationsfarbstoffe entstehen durch oxidative Kupplung aus den Oxidationsfarbstoffvorprodukten, in dem eine oder mehrere Entwicklerkomponenten untereinander oder mit einer oder mehrerer Kupplerkomponenten zum Farbstoff reagieren.

Üblicherweise eingesetzte Entwicklerkomponenten sind beispielsweise p-Phenylendiamin, p-Toluylendiamin, 2,4,5,6-Tetraaminopyrimidin, p-Aminophenol, N,N-Bis-(2-hydroxyethyl)-p-phenylendiamin, 2-(2,5-Diaminophenyl)-ethanol, 2-(2,5-Diaminophenoxy)-ethanol, 1-Phenyl-3-carboxyamido-4-amino-pyrazol-5-on, 4-Amino-3-methylphenol, 2-Aminomethyl-4-aminophenol, 2-Hydroxymethyl-4-aminophenol, 2-Hydroxy-4,5,6-triaminopyrimidin, 2,4-Dihydroxy-5,6-diaminopyrimidin, 2,5,6-Triamino-4-hydroxypyrimidin und 4,5-Diamino-1-(2-hydroxyethyl)pyrazol.

Als Kupplerkomponenten werden in der Regel m-Phenylendiaminderivate, Naphthole, Resorcin und Resorcinderivate, Pyrazolone, m-Aminophenole und substituierte Pyridinderivate verwendet. Als Kupplersubstanzen eignen sich insbesondere α-Naphthol, 1,5-, 2,7- und 1,7-Dihydroxynaphthalin, 5-Amino-2-methylphenol, 3-Aminophenol, Resorcin, Resorcinmonomethylether, 1,3-Phenylendiamin, 2,4-Diaminophenoxyethanol, 2-Amino-4-(2-hydroxyethylamino)anisol, 1-Phenyl-3-methyl-pyrazol-5-on, 2,4-Dichlor-3-aminophenol, 1,3-Bis-(2,4-diaminophenoxy)-propan, 2-Chlorresorcin, 4-Chlorresorcin, 2-Chlor-6-methyl-3-aminophenol, 2-Methylresorcin, 5-Methylresorcin, 3-Amino-6-methoxy-2-methylamino-pyridin und 3,5-Diamino-2,6-dimethoxypyridin.

Ferner können die Mittel zur oxidativen Haarfärbung zur weiteren Nuancierung direktziehende Farbstoffe enthalten. Direktziehende Farbstoffe sind üblicherweise Nitrophenylendiamine, Nitroaminophenole, Azofarbstoffe, Anthrachinone oder Indophenole. Bevorzugte direktziehende Farbstoffe sind die unter den internationalen Bezeichnungen bzw. Handelsnamen HC Yellow 2, HC Yellow 4, HC Yellow 5, HC Yellow 6, Basic Yellow 57, Disperse Orange 3, HC Red 3, HC Red BN, Basic Red 76, HC Blue 2, HC Blue 12, Disperse Blue 3, Basic Blue 99, HC Violet 1, Disperse Violet 1, Disperse Violet 4, Disperse Black 9, Basic Brown 16 und Basic Brown 17 bekannten Verbindungen sowie 1,4-Bis-(2-hydroxyethyl)-amino-2-nitrobenzol, 4-Amino-2-nitrodiphenylamin-2'-carbonsäure, 6-Nitro-1,2,3,4-tetrahydrochinoxalin, Hydroxyethyl-2-nitro-toluidin, Pikraminsäure, 2-Amino-6-chloro-4-nitrophenol.

Oxidative Färbesysteme und ihre spezifischen Ausführungsformen sind dem Fachmann wohlbekannt. In diesem Zusammenhang wird ausdrücklich auf die bekannten Monographien, z. B. Kh. Schrader, Grundlagen und Rezepturen der Kosmetika, 2. Auflage, Hüthig Buch Verlag, Heidelberg, 1989, verwiesen, die das entsprechende Wissen des Fachmannes wiedergeben. Das erfindungsgemäße Mittel wird unmittelbar vor der Anwendung aus zwei Zubereitungen, bei denen eine Zubereitung einen mineralische Säure und die andere Zubereitung mindestens ein Dithionitsalz enthalten, durch Vermischen hergestellt. Der Begriff "unmittelbar" ist dabei als ein Zeitraum von wenigen s bis maximal 5 min zu verstehen, bevorzugt weniger als 3 min und insbesondere bevorzugt weniger als 1 min zu verstehen. Dadurch können Einbußen in der Entfärbeleistung bei der Anwendung des Mittels minimiert werden.

Das anwendungsbereite Mittel weist dabei einen pH-Wert von kleiner als 5 auf (gemessen bei 25°C). Dadurch kann die Entfärbeleistung signifikant gesteigert werden. Besonders gute Entfärbeergebnisse werden erzielt, wenn der pH-Wert kleiner als 3,5, insbesondere kleiner als 2,5, weiter bevorzugt kleiner als 2,0 und insbesondere kleiner als 1,5 ist. Bevorzugt weist das Mittel jedoch einen pH-Wert von größer als 1 auf, um mögliche Reizungen der Kopfhaut minimal zu halten.

Die wässrige oder wässrig-alkoholische Zubereitung (I) enthält dabei mindestens eine mineralische Säure ausgewält aus Salzsäure und/oder Schwefelsäure. Unter einer wässrigen oder wässrig-alkoholischen Zubereitung ist dabei ein Mittel zu verstehen, welches einen wässrigen oder wässrig-alkoholischen Träger besitzt. Ein wässriger Träger enthält mindestens 50 Gew.-% Wasser. Unter wässrig-alkoholischen Trägern sind im Sinne der vorliegenden Erfindung wässrige Lösungen, enthaltend 3 bis 70 Gew.-% eines $C_1$-$C_4$-Alkohols, insbesondere Ethanol und/oder Isopropanol, zu verstehen. Die Zubereitung (I) enthält mindestens eine mineralische Säure ausgewält aus Salzsäure und/oder Schwefelsäure.

Die Zubereitung (I) enthält die mineralischen Säuren in einem Anteil, der es ermöglicht, den pH-Wert des anwendungsbereiten Mittels auf einen Wert von kleiner als 5, bevorzugt auf einen pH-Wert von kleiner als 3,5 bis größer als 1, besonders bevorzugt auf einen pH-Wert von kleiner als 2,5 bis größer als 1, weiter bevorzugt auf einen pH-Wert von kleiner als 2,0 bis größer als 1 und außerordentlich bevorzugt auf einen pH-Wert von kleiner als 1,5 bis größer als 1, einzustellen. Die durch den sauren pH-Wert aus den Dithionitsalzen entstehende dithionige Säure scheint ein zur Entfärbung geeigneteres Reduktionspotential als die entsprechenden Dithionitsalze aufzuweisen. Die Zubereitung (II) enthält mindestens ein Dithionitsalz. Erfindungsgemäß geeignete Dithionitsalze sind insbesondere Natriumdithionit ($Na_2S_2O_4$), Kaliumdithionit ($K_2S_2O_4$), und Ammoniumdithionit (($NH_4$)$_2S_2O_4$). Gemäß einer Ausführungsform des ersten Erfindungsgegenstands enthält die Zubereitung (II) als Dithionitsalz Natriumdithionit. Die Zubereitung (II) enthält Dithionitsalze dabei bevorzugt in einem solchen Anteil, dass das anwendungsbereite Mittel einen Anteil an Dithioniten von 0,1 bis 15 Gew.-%, bevorzugt von 0,5 bis 10 Gew.-% und insbesondere in einem Anteil von 1,0 bis 5,0 Gew.-%, jeweils bezogen auf das Gesamtgewicht des anwendungsbereiten Mittels, aufweist. Dadurch lassen sich insbesondere gute Entfärbeergebnisse erzielen. Gegebenenfalls können neben dem oder den Dithionitsalzen weitere Reduktionsmittel, wie beispielsweise aus DE 102006053343 A1 bekannt, in der Zubereitung (II) enthalten sein.

Eine weitere Ausführungsform des ersten Erfindungsgegenstands ist daher ein Mittel, welches dadurch gekennzeichnet ist, dass es das oder die Dithionitsalze in einem Anteil von 0,1 bis 15 Gew.-%, bevorzugt von 0,5 bis 10 Gew.-% und insbesondere in einem Anteil von 1,0 bis 5,0 Gew.-%, jeweils bezogen auf das Gesamtgewicht des anwendungsbereiten Mittels, enthält. Dies bedeutet, dass sich, sofern das anwendungsbereite Mittel mehrere Dithionitsalze enthält, die vorstehenden Mengenangaben auf den Gesamtgehalt an Dithionitsalzen beziehen.

Es hat sich gezeigt, dass die Entfärbeleistung verbessert wird, wenn das Dithionitsalz in einem molaren Überschuss zur mineralischen Säure vorliegt. Beim äquimolaren Einsatz von Dithionitsalz und Säure kann eine deutlich schwächere Entfärbung beobachtet werden. Ohne an eine Theorie gebunden zu sein, kann angenommen werden, dass durch den Überschuss laufend während des Entfärbevorgangs dithionige Säure nachgebildet wird und/oder das überschüssige Natriumdithionit ebenfalls in die Reaktion eingreift. Bevorzugt sind dabei molare Überschüsse von 2 bis 16, bevorzugt 3 bis 15 und insbesondere 4 bis 12. Auch hier beziehen sich die vorstehenden Mengenangaben auf den Gesamtgehalt an Dithionitsalzen, sofern die Zubereitung (II) mehrere Dithionitsalze enthält.

Eine weitere Ausführungsform des ersten Erfindungsgegenstands ist daher ein anwendungsbereites Mittel, welches dadurch gekennzeichnet ist, dass das molare Verhältnis in diesem anwendungsbereiten Mittel zwischen der oder den mineralischen Säuren und dem oder den Dithionitsalzen einen Wert von 2 bis 16, bevorzugt 3 bis 15 und insbesondere 4 bis 12 aufweist.

Die Zubereitung (II) kann dabei in verschiedenen kosmetischen Trägern formuliert sein. Als kosmetische Träger eignen sich besonders Cremes, Emulsionen, Gele oder auch tensidhaltige schäumende Lösungen, wie beispielsweise Shampoos oder andere Zubereitungen, die insbesondere für die Anwendung auf dem Haar geeignet sind. Es ist aber auch denkbar, die Inhaltsstoffe der Zubereitung (II) in eine pulverförmige oder auch tablettenförmige Formulierung zu integrieren, welche vor der Anwendung in Wasser oder in der Zubereitung (I) gelöst wird. In einer bevorzugten Ausführungsform der Erfindung liegt die Zubereitung (II) pulverförmig oder tablettenförmig vor.

In einer anderen bevorzugten Ausführungsform der Erfindung enthält die Zubereitung (II) mindestens ein Dithionitsalz in einer Gesamtmenge von 0,2 bis 30 Gew.-%, bevorzugt von 1,0 bis 20 Gew.-% und insbesondere in einer Gesamtmenge von 2,0 bis 10,0 Gew.-%, weiterhin 5 - 99,8 Gew.-Wasser, wobei sich alle Mengenangaben jeweils auf das Gesamtgewicht der Zubereitung (II) beziehen, und weist einen pH-Wert von 5,0 bis 7,5, bevorzugt 5,1 bis 6,5 und besonders bevorzugt 5,2 bis 6,0, auf.

Die kosmetischen Träger der Zubereitung (I) können insbesondere wässrig oder wässrig-alkoholisch sein.

Das anwendungsbereite Mittel sollte sich gut aus den beiden Zubereitungen (I) und (II) anmischen lassen. Andererseits sollte es zur Anwendung eine ausreichende Viskosität besitzen, um ein Verlaufen des Mittels vom Anwendungsort, beispielsweise aus den Strähnen menschlicher Kopfhaare zu verhindern. Dazu weisen die Mittel bevorzugt eine Viskosität von 200 bis 20000 mPa·s, insbesondere von 1500 bis 15000 mPa·s und besonders von 2000 bis 10000 mPa·s (jeweils gemessen im Brookfield-Viskosimeter RVF bei 20 rpm; 20°C) auf. "rpm" bedeutet "Rotations per minute", also Spindelumdrehungen pro Minute.

Eine weitere Ausführungsform des ersten Erfindungsgegenstands ist daher ein Mittel, welches dadurch gekennzeichnet ist, dass das Mittel eine Viskosität von 200 bis 20000 mPa·s, insbesondere von 1500 bis 15000 mPa·s und besonders von 2000 bis 10000 mPa·s (jeweils gemessen im Brookfield-Viskosimeter RVF bei 20 rpm; 20°C) aufweist.

Zur Einstellung der Viskosität kennt der Fachmann verschiedenste polymere und nichtpolymere Verdickungsmittel.

Das erfindungsgemäße Mittel enthält weiterhin mindestens ein Verdickungsmittel. Bevorzugt handelt es sich bei dem Verdickungsmittel um einen hydrophilen Verdicker. Erfindungsgemäß einsetzbare Verdickungsmittel sind unter anderem

- synthetische Polymere, insbesondere Homo- oder Co-Polymere der Acrylsäure, ihrer Salze oder ihrer Alkylester, die gegebenenfalls kationisch oder anionisch in der Alkylkette modifiziert sind; Polyacrylamidpolymere, insbesondere Homo- oder Co-Polymere von Acrylsäureamid und/oder Methacrylsäureamid, die gegebenenfalls kationisch oder anionisch modifiziert sind; und Copolymere aus Acrylsäure und Acrylsäureamid;
- polysaccharidische Polymere, wie beispielsweise Algin, Alginate, Glucane wie Dextran, Pullulan, Curdlan, Cellulose und Cellulosederivate, Laminarin, Amylose oder Lichenin, Traganth, Karaya-Gummi, Ghatti-Gummi, Agar, Carrageenan, Chitin, Chitosan, Gummi arabicum, Gellan, Carob Gummi, Galactonmannane wie Guar oder Johannisbrotkernmehl, Tamarindenkernmehl oder deren Derivate, und Xanthan;
- anorganische Verdicker, insbesondere geeignete Elektrolyte, wie Natriumchlorid oder Kaliumchlorid, Schichtsilicate (polymere, kristalline Natriumdisilicate) und Magnesium Aluminium Silicate oder Bentonite, besonders Smektite, wie Montmorillonit oder Hectorit, die gegebenenfalls auch geeignet modifiziert sein können;

sowie deren Mischungen.

Bevorzugt sind polymere Verdickungsmittel, insbesondere auf Basis von Polysacchariden.

Es hat sich gezeigt, dass insbesondere verdickende, polysaccharidische Polymere milde und lagerstabile Formulierungen ergeben. Erfindungsgemäß bevorzugte polymere Verdickungsmittel sind daher verdickende, polysaccharidische Polymere.

Als polymeres, polysaccharidisches Verdickungsmittel können beispielsweise Agar-Agar, Carrageen, Alginate, Xanthan-Gum, Karaya-Gummi, Ghatti-Gummi, Tragant, Skleroglucangums oder Gummi arabicum, Alginate, Pektine, Polyosen, Guar-Gums, Johannisbrotbaumkernmehl, Leinsamengummen, Dextrane, Pektine, Stärke-Fraktionen und Derivate wie Amylose, Amylopektin und Dextrine, Gelatine und Casein sowie Cellulosederivate verwendet werden. Besonders bevorzugte Polysaccharide sind dabei Polymere auf Cellulose-Basis. Hierbei können chemisch modifizierten Cellulosen, wie beispielsweise Acetyl-, Methyl- oder Ethylcellulosen, Carboxyalkylcellulosen wie Carboxymethylcellulose und Hydroxyalkylcellulosen wie Hydroxyethylcellulose eingesetzt werden. Solche Polymere aus den genannten Substanzklassen sind kommerziell erhältlich und werden beispielsweise unter den Handelsnamen Deuteron®-XG (anionisches Heteropolysaccharid auf Basis von $\beta$-D-Glucose, D-Manose, D-Glucuronsäure, Schoener GmbH), Deuteron®-XN (nichtionogenes Polysaccharid, Schoener GmbH), Protanal RF 6650 alginate (Natriumalginat, FMC Biopolymer), Cekol (Cellulose Gum, Kelco), Kelzan (Xanthan-Biopolymer, Kelco), Xanthan FN (Xanthan-Biopolymer, Jungbunzlauer), Keltrol z.B. Keltrol CG-T (Xanthan-Biopolymer, Kelco) oder Keltrol CG-SFT (Xanthan-Biopolymer, Kelco) angeboten.

Besonders bevorzugte Verdickungsmittel sind Xanthan, Carboxymethylcellulose und/oder Alginate. Bevorzugt kann die Kontrolle des Entfärbegrades der keratinischen Faser während der Einwirkzeit ohne Entfernen der Anwendungsmischung von der Faser visuell erfolgt. Dazu wird ein anwendungsbereites, transparentes Mittel des ersten Erfindungsgegenstands auf menschliches Haar aufgetragen, und der Aufhellprozess während der Einwirkzeit ein oder mehrmals durch visuelle Kontrolle, ohne Abtragen des Mittels von der Faser, beurteilt. Somit ist eine vereinfachte und permanente Kontrolle des Entfärbevorgangs gewährleistet.

Zur Herstellung von ausreichend viskosen, gleichzeitig aber transparenten Mitteln hat sich der Zusatz von Xanthan als Verdickungsmittel besonders bewährt. "Transparent" im Sinne der Erfindung sind solche Mittel, die bei Auftragung einer gleichmäßigen Schicht dieser Zubereitungen von 1 bis 3 mm Dicke auf einen Untergrund klar sind und so durchblickt werden können, dass das menschliche Auge die Farbe des Untergrunds ohne Trübung erkennen und beurteilen kann.

Transparenz kann vom Fachmann weiterhin durch technische Methoden gemessen werden. "Transparent" im Sinne der Erfindung sind daher auch Mittel, die bei photometrischen Messungen mit einem Methrom Photometer 662 bei 25°C optische Transmissionen von mindestens 70%, insbesondere mindestens 80%, besonders bevorzugt von mindestens 98 % aufweisen, jeweils gemessen bei einer Schichtdicke von 1 cm.

Eine Ausführungsform der vorliegenden Erfindung ist dadurch gekennzeichnet, dass das Mittel als Verdickungsmittels ein polysaccharidisches Polymer, bevorzugt Xanthan, enthält.

Erfindungsgemäß bevorzugte Mittel sind dadurch gekennzeichnet, dass das oder die Verdickungsmittel in einem Anteil von 0,05 bis 8,0 Gew.-%, insbesondere von 0,1 bis 3,0 Gew.-%, und ganz besonders von 0,5 bis 2,0 Gew.-%, jeweils bezogen auf das Gesamtgewicht des Mittels, enthalten ist.

Das polymere Verdickungsmittel kann dazu entweder nur in einer der beiden Zubereitungen (I) und (II) oder in beiden Zubereitungen eingearbeitet sein.

Das anwendungsbereite Mittel kann weiterhin zusätzlich mindestens ein organisches Lösungsmittel enthalten. Als organisches Lösungsmittel kommen die alkoholische Komponenten eines wässrig-alkoholischen Trägers der Zubereitung (I) und/oder (II) in Frage. Weiterhin können die organischen Lösungsmittel aus Polyolen, aromatischen Alkoholen sowie aromatischen und aliphatischen Ethern ausgewählt werden. Bevorzugt weisen diese Lösungsmittel eine gute Vermischbarkeit mit Wasser bei 25°C auf. Beispiele für geeignete organische Lösungsmittel sind Ethylenglycol, Propylenglycol, Butylenglycol, 1,3-Propandiol, Methoxybutanol, Benzylalkohol, Phenoxyethanol, Ethanol, n-Propanol, Isopropanol und Glycerin.

Die erfindungsgemäßen Mittel können weitere Hilfs- und Zusatzstoffe enthalten.

Weiterhin können die erfindungsgemäßen Mittel zusätzliche grenzflächenaktive Substanzen, wie anionische Tenside, zwitterionische Tenside und/oder amphotere Tenside enthalten.

Tenside sind amphiphile (bifunktionelle) Verbindungen, die aus mindestens einem hydrophoben und mindestens einem hydrophilen Molekülteil bestehen. Der hydrophobe Rest ist bevorzugt eine Kohlenwasserstoffgruppe mit 8-28 Kohlenstoff-Atomen, die gesättigt oder ungesättigt, linear oder verzweigt sein kann. Besonders bevorzugt ist diese $C_8$-$C_{28}$-Alkylgruppe linear. Tenside werden orientiert an Grenzflächen absorbiert, können zu Micellen aggregieren und bilden lyotrope Phasen, also flüssigkristalline Phasen in Lösungen, aus.

Geeignete anionische oberflächenaktive Stoffe sind gekennzeichnet durch eine wasserlöslich machende, anionische Gruppe wie beispielsweise eine Carboxylat-, Sulfat-, Sulfonat- oder Phosphat-Gruppe und eine lipophile Alkylgruppe mit 8 bis 28 C-Atomen. Insbesondere enthalten die erfindungsgemäßen Mittel zusätzlich anionische Tenside, ausgewählt aus $C_{10}$-$C_{18}$-Fettsäuren, $C_{10}$-$C_{18}$-Alkylsulfaten, sowie Alkylethersulfaten und Ethercarbonsäuren mit jeweils 10 bis 18 C-Atomen in der Alkylgruppe und bis zu 12 Glykolethergruppen im Molekül.

Die amphoteren Tenside werden in ampholytische Tenside und zwitterionische Tenside unterteilt. Unter ampholytischen Tensiden werden solche oberflächenaktiven Verbindungen verstanden, die sowohl saure (beispielsweise -COOH oder-$SO_3H$-Gruppen) als auch basische hydrophile Gruppen (beispielsweise Aminogruppen) besitzen und sich also je nach Bedingung sauer oder basisch verhalten.

Unter zwitterionischen Tensiden versteht der Fachmann Tenside, die im selben Molekül sowohl eine negative als auch eine positive Ladung tragen. Als zwitterionische Tenside bevorzugt sind solche, die im Molekül mindestens eine quartäre Ammoniumgruppe und mindestens eine Carboxylat-, Sulfonat- oder Sulfat-Gruppe tragen. Besonders geeignete zwitterionische Tenside sind die so genannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterioni- isches Tensid ist das unter der INCI-Bezeichnung Cocamidopropyl Betaine bekannte Fettsäureamid-Derivat.

Beispiele für bevorzugte ampholytische Tenside sind N-Alkylglycine, N-Alkylaminopropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils 8 bis 24 C-Atomen in der Alkylgruppe. Erfindungsgemäß bevorzugte ampholytische Tenside sind solche, die unter den INCI-Bezeichnungen Disodium Cocoamphodiacetate und Disodium Cocoamphodipropionate erhältlich sind.

Weiterhin hat es sich als vorteilhaft erwiesen, wenn die erfindungsgemäßen Mittel zusätzlich nichtionische Tenside enthalten. Solche Verbindungen sind beispielsweise Anlagerungsprodukte von 2 bis 50 Mol Ethylenoxid an $C_{12}$-$C_{30}$-Fettalkohole, $C_{12}$-$C_{30}$-Fettsäuremono- und -diester von Anlagerungsprodukten von 1 bis 30 Mol Ethylenoxid an Glycerin; Polyglycerinester und alkoxylierte Polyglycerinester, wie beispielsweise Poly(3)glycerindiisostearat und Poly(2)glycerinpolyhydroxy-stearat; alkoxylierte, bevorzugt propoxylierte und insbesondere ethoxylierte, Mono- und Diglyceride, wie beispielsweise Glycerinmonolaurat + 20 EO (Mol Ethylenoxid) und Glycerinmonostearat + 20 EO; Aminoxide; Sorbitanfettsäureester und Anlagerungeprodukte von Ethylenoxid an Sorbitanfettsäureester wie beispielsweise Polysorbate, Sorbitanmonolaurat und Sorbitanmonolaurat + 20 EO; Zuckerfettsäureester und Anlagerungsprodukte von Ethylenoxid an Zuckerfettsäureester; Anlagerungsprodukte von Ethylenoxid an Fettsäurealkanolamide und Fettamine; Fettsäure-N-alkylglucamide; Alkylphenole und Alkylphenolalkoxylate mit 6 bis 21, insbesondere 6 bis 15 Kohlenstoffatomen in der Alkylkette und 0 bis 30 Ethylenoxid- und/oder Propylenoxid-Einheiten, wie beispielsweise Nonylphenol + 4 EO, Nonylphenol + 9 EO, Octylphenol + 3 EO und Octylphenol + 8 EO, sowie Alkylpolyglycoside. Als nichtionische Tenside eignen sich insbesondere Fettalkoholethoxylate sowie Alkylmono- und -oligoglucoside und deren ethoxylierte Analoga.

Die anionischen, amphoteren, also zwitterionischen und ampholytischen, und nichtionischen Tenside sind in dem erfindungsgemäßen Mittel bevorzugt in einer Gesamtmenge von 0,05 - 30 Gew.-%, besonders bevorzugt von 0,1 - 20 Gew.-%, jeweils bezogen auf das Gesamtgewicht des Mittels, enthalten.

Weiterhin können die Mittel kationische Tenside vom Typ der quartären Ammoniumverbindungen, der Esterquats und der Amidoamine, wie Stearamidopropyldimethylamin, enthalten. Bevorzugte quaternäre Ammoniumverbindungen sind Ammoniumhalogenide, wie Alkyltrimethylammoniumchloride, Dialkyldimethylammoniu chloride und Trialkylmethylammoniumchloride sowie die unter den INCI-Bezeichnungen Quaternium-27 und Quaternium-83 bekannten Verbindungen. Weitere erfindungsgemäß verwendbare kationische Tenside stellen die quaternisierten Proteinhydrolysate dar. Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen (Warenzeichen Stepantex, Dehyquart und Armocare). Die kationischen Tenside sind in den erfindungsgemäßen Mitteln bevorzugt in Mengen von 0,05 bis 10 Gew.-%, insbesondere von 0,1 bis 5 Gew.-%, bezogen auf das gesamte Mittel, enthalten.

Ferner können die erfindungsgemäßen Mittel weitere Wirk-, Hilfs- und Zusatzstoffe enthalten, wie beispielsweise nichtionische Polymere, wie beispielsweise Vinylpyrrolidinon/Vinylacrylat-Copolymere, Polyvinylpyrrolidinon, Vinylpyrrolidinon/Vinylacetat-Copolymere, Polyethylenglykole und Polysiloxane; zusätzliche Silikone wie flüchtige oder nicht flüchtige, geradkettige, verzweigte oder cyclische, vernetzte oder nicht vernetzte Polyalkylsiloxane (wie Dimethicone oder Cyclomethicone), Polyarylsiloxane und/oder Polyalkylarylsiloxane, insbesondere Polysiloxane mit organofunktionelle Gruppen, wie substituierten oder unsubstituierten Aminen (Amodimethicone), Carboxyl-, Alkoxy- und/oder Hydroxylgruppen (Dimethiconcopolyole), lineare Polysiloxan(A)-Polyoxyalkylen(B)-Blockcopolymere, gepfropften Silikon polymere; kationische Polymere wie quaternisierte Celluloseether, Polysiloxane mit quaternären Gruppen, Dimethyldiallylammoniumchlorid-Polymere, Acrylamid-Dimethyldiallyl-ammoniumchlorid-Copolymere, mit Diethylsulfat quaternierte Dimethylamino-ethylmethacrylat-Vinylpyrrolidinon-Copolymere, Vinylpyrrolidinon-Imidazolinium-methochlorid-Copolymere und quaternierter Polyvinylalkohol, insbesondere Polyquaternium-2, Polyquaternium-4, Polyquaternium-6, Polyquaternium-7, Polyquaternium-10, Polyquaternium-11, Polyquaternium-16, Polyquaternium-24, Polyquaternium-28, Polyquaternium-37, Polyquaternium-44, Polyquaternium-46, Polyquaternium-55, Polyquaternium-67, Polyquaternium-68, Polyquaternium-69 und Polyquaternium-87; zwitterionische und amphotere Polymere, wie insbesondere Polyquaternium-22 und Polyquaternium-39; Strukturanten wie Glucose, Maleinsäure und Milchsäure, haarkonditionierende Verbindungen wie Phospholipide, beispielsweise Lecithin und Kephaline; Parfümöle, Dimethylisosorbid und Cyclodextrine; faserstrukturverbessernde Wirkstoffe, insbesondere Mono-, Di- und Oligosaccharide wie beispielsweise Glucose, Galactose, Fructose, Fruchtzucker und Lactose; Farbstoffe zum Anfärben des Mittels; Antischuppenwirkstoffe wie Piroctone Olamine, Zink Omadine und Climbazol; Aminosäuren und Oligopeptide; Proteinhydrolysate auf tierischer und/oder pflanzlicher Basis, sowie in Form ihrer Fettsäure-Kondensationsprodukte oder gegebenenfalls anionisch oder kationisch modifizierten Derivate; pflanzliche Öle; Lichtschutzmittel und UV-Blocker; Wirkstoffe wie Panthenol, Pantothensäure, Pantolacton, Allantoin, Pyrrolidinoncarbonsäuren und deren Salze sowie Bisabolol; Polyphenole, insbesondere Hydroxyzimtsäuren, 6,7-Dihydroxycumarine, Hydroxybenzoesäuren, Catechine, Tannine, Leukoanthocyanidine, Anthocyanidine, Flavanone, Flavone und Flavonole; Ceramide oder Pseudoceramide; Vitamine, Provitamine und Vitaminvorstufen; Pflanzenextrakte; Fette und Wachse wie Fettalkohole, Bienenwachs, Montanwachs und Paraffine; Quell- und Penetrationsstoffe wie Glycerin, Propylenglycolmonoethylether, Carbonate, Hydrogencarbonate, Guanidine, Harnstoffe sowie primäre, sekundäre und tertiäre Phosphate; Perlglanzmittel wie Ethylenglykolmono- und -distearat sowie PEG-3-distearat sowie Pigmente.

Die Auswahl dieser weiteren Stoffe wird der Fachmann gemäß der gewünschten Eigenschaften der Mittel treffen. Bezüglich weiterer fakultativer Komponenten sowie der eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen.

Je nach Stabilität, Löslichkeit und Vermischbarkeit der Inhaltsstoffe untereinander können die Inhaltsstoffe entweder nur in Zubereitung (I) oder nur in Zubereitung (II) oder in beiden Zubereitungen (I) und (II) eingearbeitet sein. Die beiden Zubereitungen (I) und (II) werden bevorzugt gemeinsam konfektioniert, so dass dem Anwender beide Zubereitungen im entsprechenden Verhältnis zueinander vorliegen und das erfindungsgemäße Mittel des ersten Erfindungsgegenstands durch einfaches Vermischen herstellbar ist.

Ein zweiter Erfindungsgegenstand ist daher eine Mehrkomponentenverpackungseinheit (Kit-of-Parts), welche mindestens zwei getrennt voneinander konfektionierte Container (C1) und (C2) umfasst, wobei

(i) der Container (C1) eine wässrige oder wässrig-alkoholische Zubereitung (I), enthaltend mindestens eine mineralische Säure ausgewählt aus Salzsäure und/oder Schwefelsäure, enthält, wobei die Zubereitung (I) einen pH-Wert von 0,8 - 2,5 aufweist, und

(ii) der Container (C2) zweiten Zubereitung (II), enthaltend mindestens ein Dithionitsalz, enthält

und welches dadurch gekennzeichnet ist, dass die Zubereitung (I) und/oder die Zubereitung (II) zusätzlich mindestens ein Verdickungsmittel enthält.

Die Zubereitungen der Mehrkomponentenverpackungseinheit sind in getrennt voneinander konfektionierten Containern enthalten. Unter Container wird im Rahmen der vorliegenden Erfindung eine Umhüllung verstanden, die in Form einer gegebenenfalls wieder-verschließbaren Flasche, einer Tube, einer Dose, eines Tütchens, eines Sachets oder ähnlichen Umhüllungen vorliegt. Dem Umhüllungsmaterial sind erfindungsgemäß keine Grenzen gesetzt. Bevorzugt handelt es sich jedoch dabei um Umhüllungen aus Glas oder Kunststoff.

Es kann erfindungsgemäß vorteilhaft sein, wenn die erfindungsgemäße Mehrkomponentenverpackungseinheit mindestens ein weiteres Haarbehandlungsmittel in einem getrennten Container enthält, insbesondere ein Konditioniermittel. Solche Konditioniermittel für keratinische Fasern, insbesondere Haare, sind dem Fachmann in unterschiedlichsten Formulierungen je nach spezifischem Bedürfnis und Fragestellung bekannt. Darüber hinaus kann die Verpackungseinheit Applikationshilfen, wie Kämme, Bürsten oder Pinsel, persönliche Schutzkleidung, insbesondere Ein-Weg-Handschuhe, sowie gegebenenfalls eine Gebrauchsanleitung umfassen.

Zur verbesserten Durchmischung ist vorteilhaft, wenn zumindest einer der Container eine wiederverschließbare Öffnung, wie beispielsweise einen Schnapp- oder einen Schraubverschluss, besitzt. Bevorzugt enthält mindestens eine der Zubereitungen (I) und (II) zusätzlich mindestens ein Verdickungsmittel, bevorzugt mindestens ein Polysaccharid.

Eine Ausführungsform des zweiten Erfindungsgegenstands ist daher eine Mehrkomponentenverpackungseinheit, welche dadurch gekennzeichnet ist, dass die Zubereitung (I) und/oder die Zubereitung (II) als Verdickungsmittel mindestens ein Polysaccharid, bevorzugt ausgewählt aus Xanthan, Carboxymethylcellulose und/oder Alginaten, besonders bevorzugt Xanthan, enthält. Bevorzugte Mittel enthalten in der Zubereitung (I) und/oder der Zubereitung (II) das oder die Verdickungsmittel in einem Anteil von 0,05 bis 8,0 Gew.-%, insbesondere zu 0,1 bis 3,0 Gew.-%, und ganz besonders zu 0,5 bis 2,0 Gew.-%, jeweils bezogen auf das Gesamtgewicht der jeweiligen Zubereitung.

Bevorzugt enthält mindestens eine der Zubereitungen (I) und (II) zusätzlich mindestens ein organisches Lösungsmittel, bevorzugt ausgewählt aus Ethylenglycol, Propylenglycol, Butylenglycol, 1,3-Propandiol, Methoxybutanol, Benzylalkohol, Phenoxyethanol, Ethanol, n-Propanol, Isopropanol und/oder Glycerin. Eine Ausführungsform des zweiten Erfindungsgegenstands ist daher eine Mehrkomponentenverpackungseinheit, welche dadurch gekennzeichnet ist, dass die Zubereitung (I) und/oder die Zubereitung (II) mindestens ein organisches Lösungsmittel, bevorzugt ausgewählt aus Ethylenglycol, Propylenglycol, Butylenglycol, 1,3-Propandiol, Methoxybutanol, Benzylalkohol, Phenoxyethanol, Ethanol, n-Propanol, Isopropanol und/oder Glycerin, enthält.

Bevorzugte Mittel enthalten die Zubereitung (I) und/oder (II) organische Lösungsmittel in einem Anteil von 0,1 bis 35,0 Gew.-%, insbesondere zu 0,5 bis 25,0 Gew.-%, und ganz besonders zu 1,0 bis 15,0 Gew.-%, jeweils bezogen auf das Gesamtgewicht der jeweiligen Zubereitung.

Als mineralische Säure enthält die Zubereitung (I) bevorzugt Salzsäure und/oder Schwefelsäure.

Eine Ausführungsform des zweiten Erfindungsgegenstands ist daher eine Mehrkomponentenverpackungseinheit, welche dadurch gekennzeichnet ist, dass die mineralische Säure der Zubereitung (I) ausgewählt ist aus Salzsäure und/oder Schwefelsäure.

Als Dithionitsalz enthält die Zubereitung (II) bevorzugt Natriumdithionit, Kaliumdithionit und/oder Ammoniumdithionit und insbesondere Natriumdithionit. Eine Ausführungsform des zweiten Erfindungsgegenstands ist daher eine Mehrkomponentenverpackungseinheit, welche dadurch gekennzeichnet ist, dass das Dithionitsalz der Zubereitung (II) ausgewählt ist aus Natriumdithionit, Kaliumdithionit und/oder Ammoniumdithionit, bevorzugt aus Natriumdithionit.

Hinsichtlich der weiteren bevorzugten Ausführungsführungsformen der Mehrkomponentenverpackungseinheit gelten mutatis mutandis die obigen Ausführungen des vorangehenden Erfindungsgegenstands.

Das Mittel zur reduktiven Entfärbung wird unmittelbar vor der Anwendung durch Vermischen der Zubereitungen (I) und (II) hergestellt. Anschließend wird die Zubereitung zur reduktiven Entfärbung zur Anwendung auf die oxidativ gefärbten keratinischen Fasern, insbesondere menschliche Haare, aufgetragen.

Die Erfindung betrifft daher als dritten Erfindungsgegenstand weiterhin ein Verfahren zur reduktiven Entfärbung von oxidativ gefärbten keratinischen Fasern, insbesondere menschlicher Haare, welches mehrere Verfahrensschritte umfasst:

i) Herstellung eines Mittels gemäß dem ersten Erfindungsgegenstand unmittelbar vor der Anwendung durch Vermischen von mindestens einer Zubereitung (I) und einer Zubereitung (II) aus einer Mehrkomponentenverpackungseinheit gemäß dem zweiten Erfindungsgegenstand;
ii) Aufbringen der anwendungsbereiten Zubereitung aus Verfahrensschritt i) auf die zu entfärbenden keratinischen Fasern
iii) Belassen des Mittels für einen Anwendungszeitraum von 5 bis 35 min (Minuten), bevorzugt 10 bis 30 min, weiter bevorzugt 15 bis 25 min auf den Fasern; und
iv) Ausspülen der Fasern.

Die Anwendungstemperaturen können in einem Bereich zwischen 15 und 40 °C liegen. Nach der Einwirkungszeit wird das Entfärbemittel durch Ausspülen von dem Haar entfernt. Hierzu kann entweder Wasser oder ein tensidhaltiges Mittel, wie ein Shampoo, eingesetzt werden. Das Nachwaschen mit einem Shampoo kann entfallen, wenn ein stark tensidhaltiger Träger verwendet wurde. Bevorzugt werden jedoch die Fasern im letzten Schritt mit einem handelsüblichen Shampoo behandelt und anschließend gründlich für 5 bis 15 min mit lauwarmen Wasser gespült.

Im Rahmen dieses Gegenstandes der Erfindung, das heißt, des erfindungsgemäßen Verfahrens zur reduktiven Entfärbung von oxidativ gefärbten keratinischen Fasern, gelten mutatis mutandis die voranstehend getroffenen Aussagen zu bevorzugten Ausführungsformen der erfindungsgemäßen Mittel, der erfindungsgemäßen Zubereitungen (I) und (II) sowie der erfindungsgemäßen Mehrkomponentenverpackungseinheiten analog.

Besonders bevorzugt ist ein Verfahren zur reduktiven Entfärbung von oxidativ gefärbten keratinischen Fasern, insbesondere menschlicher Haare, das dadurch gekennzeichnet ist, dass

(i) die Zubereitung (I) und die Zubereitung (II) einer Mehrkomponentenverpackungseinheit nach einem der Ansprüche 9 bis 14 miteinander zu einem anwendungsbereiten Mittel nach einem der Ansprüche 1 bis 8 vermischt werden;
(ii) das anwendungsbereite Mittel auf die zu entfärbenden Fasern aufgetragen wird;
(iii) das Mittel für einen Anwendungszeitraum von 5 bis 35 min (Minuten), bevorzugt 10 bis 30 min, weiter bevorzugt 15 bis 25 min auf den Fasern belassen wird;
(iv) und anschließend aus den Fasern ausgespült wird.

Weiter bevorzugt ist ein Verfahren zur reduktiven Entfärbung von oxidativ gefärbten keratinischen Fasern, insbesondere menschlicher Haare, das dadurch gekennzeichnet ist, dass

(i) eine wässrige oder wässrig-alkoholische Zubereitung (I), enthaltend mindestens eine mineralische Säure und Xanthan und einen pH-Wert von 0,8 - 2,5 aufweisend, mit einer Zubereitung (II), die mindestens ein Dithionitsalz in einer Gesamtmenge von 0,2 bis 30 Gew.-%, bevorzugt von 1,0 bis 20 Gew.-% und insbesondere in einer Gesamtmenge von 2,0 bis 10,0 Gew.-%, weiterhin 5 - 99,8 Gew.-Wasser, jeweils bezogen auf das Gesamtgewicht der Zubereitung (II), enthält und einen pH-Wert von 5,0 bis 7,5, bevorzugt 5,1 bis 6,5 und besonders bevorzugt 5,2 bis 6,0 aufweist, miteinander zu einem anwendungsbereiten Mittel, das einen pH-Wert von kleiner als 5, bevorzugt einen pH-Wert von kleiner als 3,5 bis größer als 1, weiter bevorzugt einen pH-Wert von kleiner als 2,5 bis größer als 1, besonders bevorzugt einen pH-Wert von kleiner als 2,0 bis größer als 1 und außerordentlich bevorzugt einen pH-Wert von kleiner als 1,5 bis größer als 1, aufweist, vermischt werden;
(ii) das anwendungsbereite Mittel auf die zu entfärbenden Fasern aufgetragen wird;
(iii) das Mittel für einen Anwendungszeitraum von 5 bis 35 min (Minuten), bevorzugt 10 bis 30 min, weiter bevorzugt 15 bis 25 min auf den Fasern belassen wird;
(iv) und anschließend aus den Fasern ausgespült wird.

Weiter bevorzugt ist ein Verfahren zur reduktiven Entfärbung von oxidativ gefärbten keratinischen Fasern, insbesondere menschlicher Haare, das dadurch gekennzeichnet ist, dass

(i) eine wässrige oder wässrig-alkoholische Zubereitung (I), enthaltend mindestens eine mineralische Säure und Xanthan und einen pH-Wert von 0,8 - 2,5 aufweisend, mit einer Zubereitung (II), die mindestens ein Dithionitsalz in einer Gesamtmenge von 0,2 bis 30 Gew.-%, bevorzugt von 1,0 bis 20 Gew.-% und insbesondere in einer Gesamtmenge von 2,0 bis 10,0 Gew.-%, weiterhin 5 - 99,8 Gew.-Wasser, jeweils bezogen auf das Gesamtgewicht der Zubereitung (II), enthält und einen pH-Wert von 5,0 bis 7,5, bevorzugt 5,1 bis 6,5 und besonders bevorzugt 5,2 bis 6,0 aufweist, miteinander zu einem anwendungsbereiten Mittel, das einen pH-Wert von kleiner als 5, bevorzugt einen pH-Wert von kleiner als 3,5 bis größer als 1, weiter bevorzugt einen pH-Wert von kleiner als 2,5 bis größer als 1, besonders bevorzugt einen pH-Wert von kleiner als 2,0 bis größer als 1 und außerordentlich bevorzugt einen pH-Wert von kleiner als 1,5 bis größer als 1, aufweist und das molare Verhältnis in diesem anwendungsbereiten Mittel zwischen der oder den mineralischen Säuren und dem oder den Dithionitsalzen einen Wert von 2 bis 16, bevorzugt 3 bis 15 und insbesondere 4 bis 12 aufweist, vermischt werden;
(ii) das anwendungsbereite Mittel auf die zu entfärbenden Fasern aufgetragen wird;
(iii) das Mittel für einen Anwendungszeitraum von 5 bis 35 min (Minuten), bevorzugt 10 bis 30 min, weiter bevorzugt 15 bis 25 min auf den Fasern belassen wird;
(iv) und anschließend aus den Fasern ausgespült wird.

Weiter bevorzugt ist ein Verfahren zur reduktiven Entfärbung von oxidativ gefärbten keratinischen Fasern, insbesondere menschlicher Haare, das dadurch gekennzeichnet ist, dass

(i) eine wässrige oder wässrig-alkoholische Zubereitung (I), enthaltend mindestens eine mineralische Säure und Xanthan und einen pH-Wert von 0,8 - 2,5 aufweisend, mit einer Zubereitung (II), die mindestens ein Dithionitsalz in einer Gesamtmenge von 0,2 bis 30 Gew.-%, bevorzugt von 1,0 bis 20 Gew.-% und insbesondere in einer Gesamtmenge von 2,0 bis 10,0 Gew.-%, weiterhin 5 - 99,8 Gew.-Wasser, jeweils bezogen auf das Gesamtgewicht der Zubereitung (II), enthält und einen pH-Wert von 5,0 bis 7,5, bevorzugt 5,1 bis 6,5 und besonders bevorzugt 5,2 bis 6,0 aufweist, miteinander zu einem anwendungsbereiten Mittel, das einen pH-Wert von kleiner als 5, bevorzugt einen pH-Wert von kleiner als 3,5 bis größer als 1, weiter bevorzugt einen pH-Wert von kleiner als 2,5 bis größer als 1, besonders bevorzugt einen pH-Wert von kleiner als 2,0 bis größer als 1 und außerordentlich bevorzugt einen pH-Wert von kleiner als 1,5 bis größer als 1, aufweist und das anwendungsbereite Mittel eine optische Transmission von mindestens 70%, insbesondere mindestens 80%, besonders bevorzugt von mindestens 98 % aufweist, jeweils gemessen bei einer Schichtdicke von 1 cm mit einem Methrom Photometer 662 bei 25°C, vermischt werden;

(ii) das anwendungsbereite Mittel auf die zu entfärbenden Fasern aufgetragen wird;

(iii) das Mittel für einen Anwendungszeitraum von 5 bis 35 min (Minuten), bevorzugt 10 bis 30 min, weiter bevorzugt 15 bis 25 min auf den Fasern belassen wird;

(iv) und anschließend aus den Fasern ausgespült wird.

Weiter bevorzugt ist ein Verfahren zur reduktiven Entfärbung von oxidativ gefärbten keratinischen Fasern, insbesondere menschlicher Haare, das dadurch gekennzeichnet ist, dass

(i) eine wässrige oder wässrig-alkoholische Zubereitung (I), enthaltend mindestens eine mineralische Säure und Xanthan und einen pH-Wert von 0,8 - 2,5 aufweisend, mit einer Zubereitung (II), die mindestens ein Dithionitsalz in einer Gesamtmenge von 0,2 bis 30 Gew.-%, bevorzugt von 1,0 bis 20 Gew.-% und insbesondere in einer Gesamtmenge von 2,0 bis 10,0 Gew.-%, weiterhin 5 - 99,8 Gew.-Wasser, jeweils bezogen auf das Gesamtgewicht der Zubereitung (II), enthält und einen pH-Wert von 5,0 bis 7,5, bevorzugt 5,1 bis 6,5 und besonders bevorzugt 5,2 bis 6,0 aufweist, miteinander zu einem anwendungsbereiten Mittel, das einen pH-Wert von kleiner als 5, bevorzugt einen pH-Wert von kleiner als 3,5 bis größer als 1, weiter bevorzugt einen pH-Wert von kleiner als 2,5 bis größer als 1, besonders bevorzugt einen pH-Wert von kleiner als 2,0 bis größer als 1 und außerordentlich bevorzugt einen pH-Wert von kleiner als 1,5 bis größer als 1, aufweist und das molare Verhältnis in diesem anwendungsbereiten Mittel zwischen der oder den mineralischen Säuren und dem oder den Dithionitsalzen einen Wert von 2 bis 16, bevorzugt 3 bis 15 und insbesondere 4 bis 12 und eine optische Transmission von mindestens 70%, insbesondere mindestens 80%, besonders bevorzugt von mindestens 98 % aufweist, jeweils gemessen bei einer Schichtdicke von 1 cm mit einem Methrom Photometer 662 bei 25°C, vermischt werden;

(ii) das anwendungsbereite Mittel auf die zu entfärbenden Fasern aufgetragen wird;

(iii) das Mittel für einen Anwendungszeitraum von 5 bis 35 min (Minuten), bevorzugt 10 bis 30 min, weiter bevorzugt 15 bis 25 min auf den Fasern belassen wird;

(iv) und anschließend aus den Fasern ausgespült wird.

In einer weiteren bevorzugten Ausführungsform sind die vorgenannten Verfahren zur reduktiven Entfärbung von oxidativ gefärbten keratinischen Fasern, insbesondere menschlicher Haare, dadurch gekennzeichnet, dass auch die Zubereitung (II) Xanthan enthält.

**Beispiele** (alle Angaben in Gew.-%)

1. Oxidationsfärbungen

Für die Beurteilung der Entfärbung wurden zunächst 12 Haarsträhnen von ca. 6 cm Länge und 0,5 g Gewicht des Codes Kerling Euronaturhaar weiß sowie Kerling 6-0 mit einem oxidativen Haarfärbemittel gefärbt. Dazu wurde folgende Färbecreme nach bekanntem Herstellungsverfahren bereitgestellt:

Farbstoffzubereitungen:

| | F1 | F2 | F3 |
|---|---|---|---|
| Cetearyl Alcohol | 6,9 | 6,6 | 12,0 |
| Fatty Alcohols, C12-18 | 2,3 | 2,4 | 2,4 |
| Ceteareth-20 | 2,0 | 0,6 | 0,9 |
| Ceteareth-12 | -- | 0,6 | -- |
| Cetrimonium Chloride (24-26%) | 3,0 | -- | -- |

(fortgesetzt)

| | F1 | F2 | F3 |
|---|---|---|---|
| Sodium Laureth (2EO) Sulfate (27%) | -- | -- | 11,0 |
| Sodium Myreth Sulfate (68-73%) | -- | 2,8 | -- |
| Cocamidopropylbetaine (29-32%) | -- | -- | 9,0 |
| Lamesoft PO 65 | -- | 2,0 | -- |
| Sodium Laureth-6 Carboxylate (21%) | -- | 10,0 | -- |
| Disodium Cocoamphodipropionate (39-41%) | -- | -- | 0,50 |
| Produkt W 37194 | -- | 3,75 | -- |
| Polyquaternium-22 (39-43%) | -- | -- | 1,00 |
| p-Toluylenediamine Sulfat | 0,73 | 0,98 | 1,01 |
| Resorcin | 0,07 | 0,49 | -- |
| 2-Methylresorcin | 0,19 | 0,02 | -- |
| 5-Amino-2-methylphenol | -- | 0,91 | 0,56 |
| 2-Amino-4-(2-hydroxyethylamino)anisol Sulfat | 0,02 | -- | -- |
| 4-Chlororesorcinol | 0,14 | 0,06 | -- |
| 2,4,5,6-Tetraaminopyrimidin Sulfat | -- | 0,03 | -- |
| 2,7-Dihydroxynaphthalen | -- | 0,14 | -- |
| 4-Amino-3-methylphenol | -- | 1,11 | -- |
| Hydroxvethyl-2-nitro-p-toluidine | -- | 0,99 | -- |
| HC RED BN | -- | -- | 0,23 |
| Serin | -- | 1,00 | -- |
| Macadamianussöl | -- | -- | 0,20 |
| Ammoniumsulfat | 0,75 | 0,41 | -- |
| Natriumsulfit | 0,50 | 0,40 | 0,20 |
| Etidronsäure (60%) | 0,20 | 0,20 | 0,20 |
| Kaliumhydroxid (50%) | -- | -- | 0,50 |
| Natriumhydroxid (45%) | -- | 2,90 | -- |
| Natriumsilicat | -- | 0,50 | 0,50 |
| Monoethanolamin | -- | -- | 2,25 |
| Ammoniak (25%) | 1,32 | 6,50 | -- |
| Schwefelsäure (20%) | -- | -- | 1,90 |
| Parfum | qs | | |
| Wasser | Ad 100 | | |

Die Ausfärbungen erfolgten nach Abmischung im Gewichtsverhältnis 1:1 mit der entsprechenden Entwicklerzubereitung E1 bis E3 zum anwendungsbereiten Färbemittel.

| | E1 | E2 | E3 |
|---|---|---|---|
| Cetyl Alkohol | 3,5 | -- | -- |
| Ceteareth-20 | 1,0 | -- | -- |

(fortgesetzt)

| | E1 | E2 | E3 |
|---|---|---|---|
| Sodium Coceth-30 Sulfate (32-34%) | 2,5 | -- | -- |
| Sodium Laureth (2EO) Sulfate (27%) | -- | 2,0 | -- |
| Emulqade F | -- | -- | 2,1 |
| Dipicolinic acid | 0,10 | 010 | -- |
| Disodium pyrophosphate | 0,03 | 0,03 | 0,03 |
| Natriumbenzoat | -- | -- | 0,04 |
| Etidronsäure (60%) | 1,5 | 1,50 | -- |
| EDTA-Na$_2$ | -- | | 0,15 |
| Dimethicone (10%) | -- | 0,07 | -- |
| Acrylates Copolymer (27-29%) | 10,0 | 15,0 | -- |
| Ammoniak (25%) | 0,65 | -- | -- |
| Isopropylpalmitat | -- | 12,1 | -- |
| Beta-Carotin | -- | 0,125 | -- |
| Marula Öl | -- | 0,125 | -- |
| Tocopherol (65%) | -- | 0,09 | -- |
| Natriumhydroxid (45%) | -- | 0,86 | -- |
| Phosphorsäure (85%) | -- | -- | 0,04 |
| Wasserstoffperoxid (50%) | 12,0 | 12,0 | 6,0 |
| Wasser | Ad 100 | | |

INCI-Bezeichnungen der Rohstoffe:

Lamesoft PO 65 (ca. 64-68% Aktivsubstanz; INCI-Bezeichnung: Coco Glucoside, Glyceryl Oleate, Aqua; BASF); Produkt W 37194 (ca. 20% Aktivsubstanz; INCI-Bezeichnung: Acrylamidopropyltrimonium Chloride/Acrylates Copolymer, Aqua; Bozzetto GmbH); Emulgade F (INCI-Bezeichnung: Cetearyl Alcohol, PEG-40 Castor Oil, Sodium Cetearyl Sulfate; BASF)

Das Gewichtsverhältnis Färbemittel : Haar betrug 4:1, die Einwirkzeit betrug 30 Minuten bei einer Temperatur von 32 Grad Celsius. Anschließend wurden die Strähnen gespült, getrocknet und mindestens 24 Stunden unter Raumbedingungen ruhen gelassen. Dann wurden die Strähnen farbmetrisch vermessen (*vide infra*).

2. Entfärbungen

2.1 Reduktive Entfärbung

Ein erfindungsgemäßes reduktives Entfärbemittel (E) wurde durch Vermischen der Zubereitungen (I) und (II) im Gewichtsverhältnis 1 : 1 hergestellt:

| | (I) | (II) |
|---|---|---|
| Xanthan Gum | 1,0 | 1,0 |
| Propylenglycol | 2,0 | 2,0 |
| Schwefelsäure (20%) | 2,0 | -- |
| Natriumdithionit | -- | 5,0 |
| Wasser | Ad 100 | |

Das anwendungsbereite Entfärbemittel (E) besitzt einen pH-Wert von 1,4. Das Entfärbemittel (E) wurde in einer Applikationsflasche vermischt, auf die Haarsträhnen bei RT appliziert und für 20 min auf den Strähnen belassen. Nach Ablauf der Einwirkzeit wurde sorgfältig mit warmem Wasser abgespült, die Haarsträhnen getrocknet und anschließend farbmetrisch vermessen.

2.2 Oxidative Entfärbung

Ein handelsübliches oxidatives Entfärbemittel/Blondiermittel (B) wurde zum Vergleich durch klumpenfreies Vermischen eines Blondierpulvers (B1) und einer Entwicklerzubereitung (B2) im Gewichtsverhältnis 1 : 2 hergestellt:

|  | (B1) |
| --- | --- |
| Natriumsilicat | 36,00 |
| Magnesiumcarbonat | 12,85 |
| Natriumhexametaphosphat | 0,20 |
| Methylmethacrylate/Methacrylic Acid Copolymer | 1,00 |
| EDTA-Na$_2$ | 0,60 |
| Polyquaternium-4 | 0,30 |
| Siliciumdioxid, hydrophil | 0,40 |
| Glycine | 0,60 |
| Kaliumpersulfat | 32,00 |
| Ammoniumpersulfat (inkl. 0,5 % Silica) | 10,00 |
| Ultramarin | 0,15 |
| Dimethicone, Dimethiconol | 1,50 |
| Paraffinum Liquidum | 3,80 |
|  |  |
|  | (B2) |
| Emulgade F | 4,00 |
| Dipicolinic acid | 0,10 |
| Disodium pyrophosphate | 0,10 |
| Natriumbenzoat | 0,04 |
| Etidronsäure (60%) | 0,30 |
| EDTA-Na$_2$ | 0,15 |
| Paraffinum Liquidum | 17,00 |
| Kaliumhydroxid (50%) | 0,24 |
| Wasserstoffperoxid (50%) | 18,20 |
| Wasser | Ad 100 |

Das Blondiermittel (B) wurde in einer Applikationsflasche klumpenfrei vermischt, auf die Haarsträhnen bei RT appliziert und für 20 min auf den Strähnen belassen. Nach Ablauf der Einwirkzeit wurde sorgfältig mit warmem Wasser abgespült, die Haarsträhnen getrocknet und anschließend farbmetrisch vermessen.

3. Entfärbeergebnisse

Die Strähnen wurden farbmetrisch mit einem Farbmessgerät der Firma Datacolor, Typ Spectraflash 450 vermessen. Die Messgrößen sind die Lab-Werte. Hierbei steht der L-Wert für die Helligkeit (je geringer der L-Wert ist, desto größer ist die Farbintensität), während der a-Wert ein Maß für den Rotanteil einer Farbe ist (d.h. je größer der a-Wert ist, umso größer ist der Rotanteil). Der b-Wert ist ein Maß für den Blauanteil der Farbe, wobei der Blauanteil umso größer ist, je negativer der b-Wert ist. Je Strähne wurden vier Messpunkte vermessen. Abschließend wurden die arithmetischen Mittel der gemessenen Lab-Werte (L*, a*, b*) gebildet.

Als wesentliches Kriterium wurde der Farbabstand dE zwischen den gefärbten und reduktiv entfärbten Strähnen herangezogen. Dieser berechnet sich aus den jeweiligen L*a*b*-Mittelwerten mittels folgender Farbabstandsformel:

$$dE = \sqrt{(L^*_{unbehandelt} - L^*_{behandelt})^2 + (a^*_{unbehandelt} - a^*_{behandelt})^2 + (b^*_{unbehandelt} - b^*_{behandelt})^2}$$

| # | Haartyp | Färbe-Behandlung | Entfärbe-Behandlung | L*-Wert | a*-Wert | b*-Wert | dE |
|---|---------|------------------|---------------------|---------|---------|---------|-----|
| 1-0 | Kerling 6-0 | unbehandelt | | 25,6 | 6,74 | 10,41 | |
| 1-1 | | F1 + E1 | -- | 18,71 | 4,41 | 2,66 | 10,63 |
| 1-1(B) | | F1 + E1 | 20 min (B) | 27,75 | 12,88 | 15,06 | 8,00 |
| 1-1(E) | | F1 + E1 | 20 min (E) | 22,42 | 6,85 | 8,47 | 3,73 |
| 1-2 | | F2 + E2 | -- | 20,14 | 9,22 | 5,49 | 7,76 |
| 1-2(B) | | F2 + E2 | 20 min (B) | 26,62 | 15,58 | 14,46 | 9,78 |
| 1-2(E) | | F2 + E2 | 20 min (E) | 26,03 | 10,23 | 13,85 | 4,92 |
| 1-3 | | F3 + E3 | -- | 16,44 | 2,6 | -0,19 | 14,61 |
| 1-3(B) | | F3 + E3 | 20 min (B) | 25,48 | 15,64 | 11,81 | 9,01 |
| 1-3(E) | | F3 + E3 | 20 min (E) | 23,86 | 7,96 | 9,87 | 2,19 |
| 2-0 | Kerling ENH weiß | unbehandelt | | 72,92 | 2,47 | 22,66 | |
| 2-1 | | F1 + E1 | -- | 22,33 | 7,80 | 3,99 | 54,19 |
| 2-1(B) | | F1 + E1 | 20 min (B) | 37,82 | 14,01 | 16,52 | 37,46 |
| 2-1(E) | | F1 + E1 | 20 min (E) | 34,45 | 8,21 | 17,42 | 39,25 |
| 2-2 | | F2 + E2 | -- | 25,84 | 17,75 | 10,08 | 51,07 |
| 2-2(B) | | F2 + E2 | 20 min (B) | 35,12 | 18,69 | 18,48 | 41,34 |
| 2-2(E) | | F2 + E2 | 20 min (E) | 47,28 | 15,15 | 27,79 | 29,06 |
| 2-3 | | F3 + E3 | -- | 16,38 | 6,89 | -2,08 | 61,87 |
| 2-3(B) | | F3 + E3 | 20 min (B) | 28,77 | 18,02 | 10,42 | 48,38 |
| 2-3(E) | | F3 + E3 | 20 min (E) | 42,63 | 10,56 | 22,79 | 31,35 |

Es zeigt sich, dass die erfindungsgemäßen Entfärbemittel behandelten Strähnen jeweils einen kleineren Farbabstand (dE) zum unbehandelten Haar als die blondierten Strähnen aufweisen und damit einen Farbton näher am ursprünglichen Haarfarbe erzielten. Auch visuelle Begutachtung der einzelnen Strähnen führte zu dem Ergebnis, dass die erfindungsgemäß entfärbten Strähnen der Ursprungshaarfarbe in Intensität und Ton deutlich näher waren als die blondierten Strähnen.

4. Haarschädigungsuntersuchungen mittels NIR-Analytik

Haare können durch oxidative Behandlung wie oxidative Färbung geschädigt werden. Cystin ist ein Bestandteil des menschlichen Haares. Cystin zeigt bei einer Wellenlänge von 6200 cm$^{-1}$ bis 5500 cm$^{-1}$ typische Schwingungen im NIR-Spektrum (Nah-Infra-Rot). Die in Cystin enthaltene DisulfidBrücke kann bei einer Haarbehandlung oxidativ gebrochen werden, und der resultierende Thiol durch weitere Oxidation in die Sulfonsäure-Gruppe der Cysteinsäure überführt. Somit verändert sich das Haar hinsichtlich seines Cysteinsäuregehaltes, welcher mit stärkerer, oxidativer Schädigung zunimmt. Cysteinsäure zeigt sich im NIR-Spektrum bei einer Wellenlänge von 5020 cm$^{-1}$ bis 4020 cm$^{-1}$.

Die Abnahme an Cystin und die Zunahme an Cysteinsäure stellt daher einen Indikator für die Schädigung des Haares dar, die mit NIR-Analysen bestimmt werden kann. Eine detaillierte Beschreibung der Methode findet sich in Evaluation of Physical Properties of Human Hairby Diffuse Reflectance Near-Infrared Spectroscopy, Y. Miyamae, Y. Yamakawa und Y. Ozaki in Applied Spectroscopy, 2007, Vol. 61 (2), S. 212 ff.

Nach Mittelwertbildung aus jeweils 9 unabhängigen Messungen wurde an voranstehend beschriebenen Strähnen von Kerling 6-0 die jeweilige Cysteinsäure-Menge und damit der Schädigungsgrad der Strähnen ermittelt.

|  | NIR-Analysenwert [mol Cysteinsäure / 100 mol Aminosäure] |
|---|---|
| 1-0 | **0,6** |
| 1-1 | **1,1** |
| 1-1(B) | **2,9** |
| 1-1(E) | **2,0** |
| 1-2 | **0,9** |
| 1-2(B) | **3,3** |
| 1-2(E) | **1,7** |
| 1-3 | **0,7** |
| 1-3(B) | **3,3** |
| 1-3(E) | **1,7** |

Die Strähnen, welche mit dem erfindungsgemäßen Entfärbemittel entfärbt wurden, (1-1/2/3(E)) wiesen jeweils einen deutlich verringerten Cysteinsäureanteil auf als die oxidativ entfärbten Strähnen (1-1/2/3(B)) und waren damit signifikant weniger geschädigt.

## Patentansprüche

1. Anwendungsbereites Mittel zum Farbabzug von oxidativ gefärbten keratinischen Fasern, welches unmittelbar vor der Anwendung durch Mischen einer ersten wässrigen oder wässrig-alkoholischen Zubereitung (I), enthaltend mindestens eine mineralische Säure ausgewählt aus Salzsäure und/oder Schwefelsäure, mit einer zweiten Zubereitung (II), enthaltend mindestens ein Dithionitsalz, erhalten wird, **dadurch gekennzeichnet, dass** das anwendungsbereite Mittel einen pH-Wert von kleiner als 5 aufweist und zusätzlich ein Verdickungsmittel enthält.

2. Anwendungsbereites Mittel nach Anspruch 1, **dadurch gekennzeichnet, dass** es das oder die Dithionitsalze in einem Anteil von 0,1 bis 15 Gew.-%, bevorzugt von 0,5 bis 10 Gew.-% und insbesondere in einem Anteil von 1,0 bis 5,0 Gew.-%, jeweils bezogen auf das Gesamtgewicht des anwendungsbereiten Mittels, enthält.

3. Anwendungsbereites Mittel nach einem der Ansprüche 1 oder 2, **dadurch gekennzeichnet, dass** das molare Verhältnis in diesem anwendungsbereiten Mittel zwischen der oder den mineralischen Säuren und dem oder den Dithionitsalzen einen Wert von 2 bis 16, bevorzugt 3 bis 15 und insbesondere 4 bis 12 aufweist.

4. Anwendungsbereites Mittel nach einem der Ansprüche 1 bis 3, **dadurch gekennzeichnet, dass** das anwendungsbereite Mittel eine Viskosität von 200 bis 20000 mPa·s, insbesondere von 1500 bis 15000 mPa·s und besonders von 2000 bis 10000 mPa·s (jeweils gemessen im Brookfield-Viskosimeter RVF bei 20 rpm; 20°C) aufweist.

5. Anwendungsbereites Mittel nach einem der Ansprüche 1 bis 4, **dadurch gekennzeichnet, dass** das anwendungsbereite Mittel einen pH-Wert von kleiner als 3,5 bis größer als 1, bevorzugt einen pH-Wert von kleiner als 2,5 bis größer als 1, besonders bevorzugt einen pH-Wert von kleiner als 2,0 bis größer als 1 und außerordentlich bevorzugt einen pH-Wert von kleiner als 1,5 bis größer als 1, aufweist.

6. Anwendungsbereites Mittel nach einem der Ansprüche 1 bis 5, **dadurch gekennzeichnet, dass** die Zubereitung (II) pulverförmig oder tablettenförmig vorliegt.

7. Anwendungsbereites Mittel nach einem der Ansprüche 1 bis 5, **dadurch gekennzeichnet, dass** die Zubereitung (II) mindestens ein Dithionitsalz in einer Gesamtmenge von 0,2 bis 30 Gew.-%, bevorzugt von 1,0 bis 20 Gew.-% und insbesondere in einer Gesamtmenge von 2,0 bis 10,0 Gew.-%, weiterhin 5 - 99,8 Gew.-Wasser, jeweils bezogen auf das Gesamtgewicht der Zubereitung (II), enthält und einen pH-Wert von 5,0 bis 7,5, bevorzugt 5,1 bis 6,5 und besonders bevorzugt 5,2 bis 6,0 aufweist.

8. Anwendungsbereites Mittel nach einem der Ansprüche 1 bis 7, **dadurch gekennzeichnet, dass** das anwendungsbereite Mittel eine optische Transmission von mindestens 70%, insbesondere mindestens 80%, besonders bevorzugt von mindestens 98 % aufweist, jeweils gemessen bei einer Schichtdicke von 1 cm mit einem Methrom Photometer 662 bei 25°C.

9. Mehrkomponentenverpackungseinheit (Kit-of-Parts), umfassend mindestens zwei getrennt voneinander konfektionierte Container (C1) und (C2), wobei

   (i) der Container (C1) eine wässrige oder wässrig-alkoholische Zubereitung (I), enthaltend mindestens eine mineralische Säure ausgewählt aus Salzsäure und/oder Schwefelsäure, enthält, wobei die Zubereitung (I) einen pH-Wert von 0,8 bis 2,5 aufweist, und
   (ii) der Container (C2) eine zweite Zubereitung (II), enthaltend mindestens ein Dithionitsalz, enthält, **dadurch gekennzeichnet, dass** die Zubereitung (I) und/oder die Zubereitung (II) zusätzlich mindestens ein Verdickungsmittel enthält.

10. Mehrkomponentenverpackungseinheit nach Anspruch 9, **dadurch gekennzeichnet, dass** die Zubereitung (I) und/oder die Zubereitung (II) als zusätzliches Verdickungsmittel mindestens ein Polysaccharid, bevorzugt ausgewählt aus Xanthan, Carboxymethylcellulose und/oder Alginaten, enthält.

11. Mehrkomponentenverpackungseinheit nach einem der Ansprüche 9 oder 10, **dadurch gekennzeichnet, dass** die Zubereitung (I) und/oder die Zubereitung (II) zusätzlich mindestens ein organisches Lösungsmittel, bevorzugt ausgewählt aus Ethylenglycol, Propylenglycol, Butylenglycol, 1,3-Propandiol, Methoxybutanol, Benzylalkohol, Phenoxyethanol, Ethanol, n-Propanol, Isopropanol und/oder Glycerin, enthält.

12. Mehrkomponentenverpackungseinheit nach einem der Ansprüche 9 bis 11, **dadurch gekennzeichnet, dass** das anwendungsbereite Mittel, das unmittelbar vor der Anwendung durch Mischen der Zubereitung (I) mit der Zubereitung (II) erhalten wird, einen pH-Wert von kleiner als 5, bevorzugt einen pH-Wert von kleiner als 3,5 bis größer als 1, besonders bevorzugt einen pH-Wert von kleiner als 2,5 bis größer als 1, weiter bevorzugt einen pH-Wert von kleiner als 2,0 bis größer als 1 und außerordentlich bevorzugt einen pH-Wert von kleiner als 1,5 bis größer als 1, aufweist.

13. Mehrkomponentenverpackungseinheit nach einem der Ansprüche 9 bis 12, **dadurch gekennzeichnet, dass** die Zubereitung (II) pulverförmig oder tablettenförmig vorliegt.

14. Verfahren zur reduktiven Entfärbung oxidativ gefärbter keratinischer Fasern, **dadurch gekennzeichnet, dass**

   (iii) die Zubereitung (I) und die Zubereitung (II) einer Mehrkomponentenverpackungseinheit nach einem der Ansprüche 9 bis 13 miteinander zu einem anwendungsbereiten Mittel nach einem der Ansprüche 1 bis 8 vermischt werden;
   (iv) das anwendungsbereite Mittel auf die zu entfärbenden Fasern aufgetragen wird;
   (v) das Mittel für einen Anwendungszeitraum von 5 bis 35 min, bevorzugt 10 bis 30 min, weiter bevorzugt 15 bis 25 min auf den Fasern belassen wird;
   (vi) und anschließend aus den Fasern ausgespült wird.

**Claims**

1. A ready-to-apply agent for removing color from oxidatively dyed keratin fibers, which agent is obtained immediately before application by mixing a first aqueous or aqueous-alcoholic preparation (I) containing at least one mineral acid selected from hydrochloric acid and/or sulfuric acid with a second preparation (II) containing at least one dithionite salt, **characterized in that** the ready-to-apply agent has a pH of less than 5 and additionally contains a thickener.

2. The ready-to-apply agent according to claim 1, **characterized in that** it contains the dithionite salt(s) in a proportion of 0.1 to 15 wt.%, preferably 0.5 to 10 wt.%, and in particular in a proportion of 1.0 to 5.0 wt.%, based in each case on the total weight of the ready-to-apply agent.

3. The ready-to-apply agent according to either claim 1 or claim 2, **characterized in that** the molar ratio in this ready-to-apply agent between the mineral acid(s) and the dithionite salt(s) has a value of 2 to 16, preferably 3 to 15, and in particular 4 to 12.

4. The ready-to-apply agent according to one of claims 1 to 3, **characterized in that** the ready-to-apply agent has a viscosity of 200 to 20000 mPa·s, in particular 1500 to 15000 mPa·s, and particularly 2000 to 10000 mPas (measured in each case using a RVF Brookfield viscometer at 20 rpm; 20 °C).

5. The ready-to-apply agent according to one of claims 1 to 4, **characterized in that** the ready-to-apply agent has a pH of less than 3.5 to greater than 1, preferably a pH of less than 2.5 to greater than 1, particularly preferably a pH of less than 2.0 to greater than 1, and most particularly preferably a pH of less than 1.5 to greater than 1.

6. The ready-to-apply agent according to one of claims 1 to 5, **characterized in that** the preparation (II) is present in powdered or tablet form.

7. The ready-to-apply agent according to one of claims 1 to 5, **characterized in that** the preparation (II) contains at least one dithionite salt in a total amount of 0.2 to 30 wt.%, preferably 1.0 to 20 wt.%, and in particular in a total amount of 2.0 to 10.0 wt.%, and furthermore 5 - 99.8 wt. water, based in each case on the total weight of the preparation (II), and has a pH of 5.0 to 7.5, preferably 5.1 to 6.5 and particularly preferably 5.2 to 6.0.

8. The ready-to-apply agent according to one of claims 1 to 7, **characterized in that** the ready-to-apply agent has an optical transmission of at least 70%, in particular at least 80%, particularly preferably at least 98%, measured in each case at a layer thickness of 1 cm and using a Methrom 622 Photometer at 25°C.

9. A kit of parts comprising at least two separately packaged containers (C1) and (C2),

   (i) the container (C1) containing an aqueous or aqueous-alcoholic preparation (I) containing at least one mineral acid selected from hydrochloric acid and/or sulfuric acid, the preparation (I) having a pH of 0.8 to 2.5, and
   (ii) the container (C2) containing a second preparation (II) containing at least one dithionite salt, **characterized in that** the preparation (I) and/or the preparation (II) additionally contains at least one thickener.

10. The kit of parts according to claim 9, **characterized in that** the preparation (I) and/or the preparation (II) contains at least one polysaccharide, preferably selected from xanthan gum, carboxymethyl cellulose and/or alginates, as the additional thickener.

11. The kit of parts according to either claim 9 or claim 10, **characterized in that** the preparation (I) and/or the preparation (II) additionally contains at least one organic solvent, preferably selected from ethylene glycol, propylene glycol, butylene glycol, 1,3-propanediol, methoxybutanol, benzyl alcohol, phenoxyethanol, ethanol, n-propanol, isopropanol and/or glycerol.

12. The kit of parts according to one of claims 9 to 11, **characterized in that** the ready-to-apply agent obtained immediately before application by mixing the preparation (I) with the preparation (II) has a pH of less than 5, preferably a pH of less than 3.5 to greater than 1, particularly preferably a pH of less than 2.5 to greater than 1, more preferably a pH of less than 2.0 to greater than 1, and most particularly preferably a pH of less than 1.5 to greater than 1.

13. The kit of parts according to one of claims 9 to 12, **characterized in that** the preparation (II) is present in powdered or tablet form.

14. A method for reductive decoloration of oxidatively dyed keratin fibers, **characterized in that**

   (iii) the preparation (I) and the preparation (II) of a kit of parts according to one of claims 9 to 13 are mixed together to form a ready-to-apply agent according to one of claims 1 to 8;
   (iv) the ready-to-apply agent is applied to the fibers to be decolored;
   (v) the agent is left on the fibers for an application time of 5 to 35 min, preferably 10 to 30 min, more preferably 15 to 25 min;

(vi) and is subsequently rinsed out of the fibers.

**Revendications**

1. Agent prêt à l'emploi pour la décoloration de fibres kératiniques teintes par oxydation, lequel est obtenu juste avant l'emploi par le mélange d'une première préparation aqueuse ou hydroalcoolique (I), contenant au moins un acide minéral choisi entre l'acide chlorhydrique et/ou l'acide sulfurique, et d'une deuxième préparation (II), contenant au moins un sel dithionite, **caractérisé en ce que** l'agent prêt à l'emploi présente une valeur de pH inférieure à 5 et contient en plus un agent épaississant.

2. Agent prêt à l'emploi selon la revendication 1 **caractérisé en ce qu'**il contient le ou les sel(s) dithionite(s) dans une proportion de 0,1 à 15 % en poids, de préférence de 0,5 à 10 % en poids et en particulier dans une proportion de 1,0 à 5,0 % en poids, par rapport au poids total de l'agent prêt à l'emploi respectivement.

3. Agent prêt à l'emploi selon une des revendications 1 ou 2 **caractérisé en ce que** le rapport molaire entre le ou les acides minéraux et le ou les sels dithionites, dans cet agent prêt à l'emploi, a une valeur de 2 à 16, de préférence de 3 à 15 et en particulier de 4 à 12.

4. Agent prêt à l'emploi selon l'une quelconque des revendications 1 à 3 **caractérisé en ce que** l'agent prêt à l'emploi présente une viscosité de 200 à 20000 mPa·s, en particulier de 1500 à 15000 mPa·s et en particulier de 2000 à 10000 mPa·s (mesuré avec le viscosimètre Brookfield RVF à 20 rpm ; 20 °C).

5. Agent prêt à l'emploi selon l'une quelconque des revendications 1 à 4 **caractérisé en ce que** l'agent prêt à l'emploi présente une valeur de pH inférieure à 3,5 et supérieure à 1, de préférence une valeur de pH inférieure à 2,5 et supérieure à 1, de manière particulièrement préférée une valeur de pH inférieure à 2,0 et supérieure à 1 et de manière préférée entre toutes une valeur de pH inférieure à 1,5 et supérieure à 1.

6. Agent prêt à l'emploi selon l'une quelconque des revendications 1 à 5 **caractérisé en ce que** la préparation (II) se présente sous forme de poudre ou de comprimés.

7. Agent prêt à l'emploi selon l'une quelconque des revendications 1 à 5 **caractérisé en ce que** la préparation (II) contient au moins un sel dithionite dans une quantité totale de 0,2 à 30 % en poids, de préférence de 1,0 à 20 % en poids et en particulier dans une quantité totale de 2,0 à 10,0 % en poids, ainsi que 5 à 99,8 % en poids d'eau, par rapport au poids total de la préparation (II) respectivement, et présente une valeur de pH de 5,0 à 7,5, de préférence de 5,1 à 6,5 et de manière particulièrement préférée de 5,2 à 6,0.

8. Agent prêt à l'emploi selon l'une quelconque des revendications 1 à 7 **caractérisé en ce que** l'agent prêt à l'emploi présente une transmission optique d'au moins 70 %, en particulier d'au moins 80 %, de manière particulièrement préférée d'au moins 98 %, mesurée respectivement pour une épaisseur de couche de 1 cm avec un photomètre de Methrom modèle 662 à 25 °C.

9. Unité de conditionnement multi-composant (kit d'éléments), comprenant au moins deux réservoirs (C1) et (C2) réalisés séparément l'un de l'autre, dans laquelle

   (i) le réservoir (C1) contient une préparation aqueuse ou hydro-alcoolique (I), contenant au moins un acide minéral choisi entre l'acide chlorhydrique et/ou l'acide sulfurique, la préparation (I) présentant une valeur de pH de 0,8 à 2,5, et
   (ii) le réservoir (C2) contient une deuxième préparation (II), contenant au moins un sel dithionite, **caractérisé en ce que** la préparation (I) et/ou la préparation (II) contient en plus au moins un agent épaississant.

10. Unité de conditionnement multi-composant selon la revendication 9 **caractérisée en ce que** la préparation (I) et/ou la préparation (II) contiennent au moins un polysaccharide, choisi de préférence parmi le xanthane, la carboxyméthylcellulose et/ou les alginates, comme agent épaississant supplémentaire.

11. Unité de conditionnement multi-composant selon une des revendications 9 ou 10 **caractérisée en ce que** la préparation (I) et/ou la préparation (II) contiennent en plus au moins un solvant organique, choisi de préférence parmi l'éthylène glycol, le propylène glycol, le butylène glycol, l'1,3-propanediol, le méthoxybutanol, l'alcool benzylique, le phénoxyéthanol, l'éthanol, le n-propanol, l'isopropanol et/ou la glycérine.

**12.** Unité de conditionnement multi-composant selon l'une quelconque des revendications 9 à 11 **caractérisée en ce que** l'agent prêt à l'emploi est obtenu juste avant l'emploi par le mélange de la préparation (I) et de la préparation (II), **en ce qu'**il présente une valeur de pH inférieure à 5, de préférence une valeur de pH inférieur à 3,5 et supérieure à 1, de manière particulièrement préférée une valeur de pH inférieure à 2,5 et supérieure à 1, de manière davantage préférée une valeur de pH inférieure à 2,0 et supérieure à 1 et de manière préférée entre toutes une valeur de pH inférieure à 1,5 et supérieure à 1.

**13.** Unité de conditionnement multi-composant selon l'une quelconque des revendications 9 à 12 **caractérisée en ce que** la préparation (II) se présente sous forme de poudre ou de comprimés.

**14.** Procédé de décoloration réductive de fibres kératiniques teintes par oxydation, **caractérisé en ce que**

(iii) la préparation (I) et la préparation (II) d'une unité de conditionnement multi-composant selon l'une quelconque des revendications 9 à 13 sont mélangées l'une avec l'autre pour donner un agent prêt à l'emploi selon l'une quelconque des revendications 1 à 8 ;
(iv) l'agent prêt à l'emploi est appliqué sur les fibres à décolorer;
(v) l'agent est laissé sur les fibres pendant une durée d'application de 5 à 35 min, de préférence de 10 à 30 min, de manière davantage préférée de 15 à 25 min ;
(vi) et est ensuite enlevé des fibres par rinçage.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1300136 A2
- DE 102006053402 A1
- DE 10152940 A
- DE 102006053343 A1

**In der Beschreibung aufgeführte Nicht-Patentliteratur**

- **KH. SCHRADER.** Grundlagen und Rezepturen der Kosmetika. Hüthig Buch Verlag, 1989
- **Y. MIYAMAE ; Y. YAMAKAWA ; Y. OZAKI.** Evaluation of Physical Properties of Human Hairby Diffuse Reflectance Near-Infrared Spectroscopy. *Applied Spectroscopy,* 2007, vol. 61 (2), 212 ff